# EUROPEAN PATENT SPECIFICATION

(11) **EP 1 769 677 B1**
(45) Date of publication and mention of the grant of the patent: **04.11.2009**
(21) Application number: 05740634.0
(22) Date of filing: 21.04.2005
(51) Int. Cl.: A01K 67/027

(54) **METHOD OF IDENTIFYING THERAPEUTIC COMPOUNDS WHICH CAN BE USED FOR THE TREATMENT AND/OR PREVENTION OF INFECTIONS AND DISEASES CAUSED BY HUMAN HERPESVIRUSES**
VERFAHREN ZUR IDENTIFIZIERUNG THERAPEUTISCHER VERBINDUNGEN, DIE SICH ZUR BEHANDLUNG UND/ODER VORBEUGUNG VON DURCH MENSCHLICHE HERPESVIREN VERURSACHTEN INFEKTIONEN UND KRANKHEITEN VERWENDEN LASSEN
METHODE PERMETTANT D'IDENTIFIER DES COMPOSES UTILES SUR LE PLAN THERAPEUTIQUE AFIN DE TRAITER ET/OU DE PREVENIR DES INFECTIONS ET DES MALADIES CAUSEES PAR LES HERPESVIRUS HUMAINS

(30) Priority: 21.04.2004 ES 200400965 P
(43) Date of publication of application: 04.04.2007
(73) Proprietor: Universidad Aut Noma de Madrid, E-28049 Madrid (ES); Consejo Superior de Investigaciones Cientificas, 28006 Madrid (ES)
(72) Inventor: VALDIVIESO ÁMATE,Fernando, Ciudad Universitaria de Cantoblanco, E-28049 Madrid (ES); BURGOS MUÑOZ, Javier S., Ciudad Universitaria de Cantoblanco, E-28049 Madrid (ES); RAMÍREZ MORENO, Carlos, Ciudad Universitaria de Cantoblanco, E-28049 Madrid (ES); SASTRE MERLÍN, Isabel, E-28006 Madrid (ES)
(74) Representative: ABG Patentes, S.L.
(86) International application number: PCT/ES2005/000209
(87) International publication number: WO 2005/101956

(56) References cited:
- KURATA T ET AL: 'Vertical transmissions of type 2 Herpes Simplex Virus infection in immuno suppresed hamsters.' JAPANESE JOURNAL OF EXPERIMENTAL MEDICINE. vol. 48, no. 5, 1978, pages 437 - 443, XP008067392
- BRAVO FJ ET AL: 'An animal model of neonatal cytomegalovirus infection.' ANTIVIRAL RESEARCH. vol. 60, no. 1, September 2003, pages 41 - 49, XP003007923
- BOURNE N ET AL: 'Preconception Immunization with a Cytomegalovirus (CMV) Glycoprotein Vaccine Improves Pregnancy Outcome in a Guinea Pig Model of Congenital CMV Infection.' JOURNAL OF INFECTIOUS DISEASES. vol. 183, 2001, pages 59 - 64, XP008076406
- JENNINGS R ET AL: 'Evaluation of a novel, anti-herpes simplex virus compound, acyclovir elaidate (P-4010), in the female guinea pig model of genital herpes.' ANTIMICROBIAL AGENTS AND CHEMOTHERAPY. vol. 43, no. 1, January 1999, pages 53 - 61, XP003007924
- SCHLEISS MR ET AL: 'Quantitative-competitive PCR monitoring of viral load following experimental guinea pig cytomegalovirus infection.' JOURNAL OF VIROLOGICAL METHODS. vol. 108, no. 1, March 2003, pages 103 - 110, XP003007925
- MEYOHAS MARIE-CAROLINE ET AL: 'Study of mother-to-child Epstein-Barr virus transmission by means of nested PCRs.' JOURNAL OF VIROLOGY. vol. 70, no. 10, 1996, pages 6816 - 6819, XP003007926
- TANG J ET AL: "Building a mouse model hallmarking the congenital human cytomegalovirus infection in central nervous system" ARCH VIROL, vol. 147, 2002, pages 1189-1195,

### SEQUENCE LISTING

<110> UNIVERSIDAD AUT6NOMA DE MADRID
   <110> CONSEJO SUPERIOR DE INVESTIGACIONES CIENTÍFICAS
<120> METHOD FOR IDENTIFYING COMPOUNDS THAT ARE THERAPEUTICALLY USEFUL FOR TREATING AND/OR PREVENTING INFECTIONS AND DISEASES CAUSED BY HUMAN HERPESVIRUSES
<130> P1511PC00
<150> ES 200400965
   <151> 2004-04-21
<160> 6
<170> PatentIn version 3.1
<210> 1
   <211> 30
   <212> DNA
   <213> Artificial sequence
<220> Synthetic DNA
   <223> Primer designed for amplifying, in combination with SEQ ID NO: 2, a 379 bp fragment of the β-actin gene (PCR positive control)
<400> 1
   aaccctaagg ccaaccgtga aaagatgacc 30
<210> 2
   <211> 22
   <212> DNA
   <213> Artificial sequence
<220> Synthetic DNA
   <223> Primer designed for amplifying, in combination with SEQ ID NO: 1, a 379 bp fragment of the β-actin gene (PCR positive control)
<400> 2
   ccagggagga agaggatgcg gc 22
<210> 3
   <211> 20
   <212> DNA
   <213> Artificial sequence
<220> Synthetic DNA
   <223> Primer designed for amplifying, in combination with SEQ ID NO: 4, a 120 bp fragment of the DNA polymerase gene (pol) of HSV-1
<400> 3
   ggtgaacgtc ttttcgcact 20
<210> 4
   <211> 20
   <212> DNA
   <213> Artificial sequence
<220> Synthetic DNA
   <223> Primer designed for amplifying, in combination with SEQ ID NO: 3, a 120 bp fragment of the DNA polymerase gene (pol) of HSV-1
<400> 4
   gtgttgtgcc gcggtctcac 20
<210> 5
   <211> 19
   <212> DNA
   <213> Artificial sequence
<220> Synthetic DNA
   <223> Primer designed for amplifying, in combination with SEQ ID NO: 6, a 110 bp fragment of the timidine kinase gene (TK) of HSV-1
<400> 5
   ataccgacga tctgcacct 19
<210> 6
   <211> 19
   <212> DNA
   <213> Artificial sequence
<220> Synthetic DNA
   <223> Primer designed for amplifying, in combination with SEQ ID NO: 5, a 110 bp fragment of the timidine kinase gene (TK) of HSV-1
<400> 6
   ttattgccgt gcgg 14

## Description

### FIELD OF THE INVENTION

The invention relates to a method for identifying compounds that are potentially useful for preventing and/or treating infections and diseases caused by human herpesviruses comprising the use of an animal model developed from the vertical transmission of human herpesviruses in said animal model.

### BACKGROUND OF THE INVENTION

Herpesviruses are members of the *Herpesviridae* family. These viruses are large, have a double-strand DNA (dsDNA) genome of about 80-250 kilobases (kb) and are found in a wide range of host systems. About 100 herpesviruses have been isolated in various animal species, including the human species.

Eight human herpesviruses have been described until now: herpes simplex virus type 1 (HSV-1), herpes simplex virus type 2 (HSV-2), varicella-zoster virus (VZV), cytomegalovirus (CMV), human herpesvirus 6 (HHV-6), human herpesvirus 7 (HHV-7), Epstein-Barr virus (EBV) and Kaposi's herpesvirus (HHV-8). These human herpesviruses are in turn grouped into various sub-families. The members of the alpha-human herpesvirus sub-family (HSV-1, HSV-2 and VZV) are neurotropic whereas the members of the gamma-human herpesvirus sub-family (EBV and HHV-8) are lymphotropic. CMV, HHV-6 and HHV-7 belong to the beta-human herpesvirus sub-family. Each one of said human herpesviruses is related to a human disease, for example labial and genital herpes labial (HSV-1 and HSV-2), varicella (VZV), infectious mononucleosis and nasopharyngeal carcinoma (EBV), pneumonia and retinitis (CMV), sudden exanthema (HHV-6 and HHV-7) and Kaposi's sarcoma (HHV-8).

After the primary infection, usually in childhood, herpesviruses establish latency in the specific host cells of the infected individual and remain there for the rest of the individual's life, possibly causing secondary infections on occasions. Primary infections with herpesviruses often differ in their clinical symptoms from secondary or recurring infections. Both primary and recurring herpesvirus infections may infect the central nervous system (CNS) and cause a disease at any time. In fact, HSV-1, a neurotropic virus infecting about 90% of the adult population worldwide, is the most common virus infecting nervous tissue in neonates, children and adults associated with various neurological diseases and neuropathological disorders, such as severe human encephalitis, Alzheimer's disease, boxer's dementia, dementia associated with the human immunodeficiency virus (HIV) and cerebral paralysis. After the primary infection, HSV-1 reaches the CNS by means of reverse axonal transport and establishes a latent state. The virus may subsequently be reactivated in response to different stimuli and reach the primary infection area by means of anterograde transport, causing recurring mucocutaneous lesions.

Although some clinical manifestations caused by human herpesviruses are not malignant, there are pathologies which potentially compromise the individuals life, such as encephalitis and generalized neonatal infections. In fact, encephalitis caused by HSV-1 and VZV has been widely described and has a yearly incidence of about 1 to 4 cases out of a million people. The absence of suitable treatment may result in a fatal outcome or may leave severe sequelae. On the other hand, neonatal encephalitis caused by human herpesviruses is a devastating disease which normally occurs as a result of perinatal HSV-2 transmission. In immunocompetent patients over the age of 3 months, meningitis, meningoencephalitis and myelitis have often been associated with HSV-2.

Treatment of infections caused by human herpesviruses is usually done with antiviral agents, such as acyclovir, cidofovir, famcyclovir, ganciclovir, penciclovir, valacyclovir or foscarnet, acyclovir being the drug of choice in the prevention and treatment of human systemic herpesvirus infections. Nevertheless, these drugs generally have a relatively low bioavailability and are potentially toxic.

Although there are antiviral drugs for preventing and treating infections and diseases caused by human herpesviruses, it is still necessary to find new compounds that are useful for treating and/or preventing infections caused by said viruses. In this sense, animal models susceptible of being used in research related to human herpesviruses would be a valuable tool for evaluating compounds that are potentially useful in treating and/or preventing infections and diseases caused by human herpesviruses.

Tang et al. (2002) Arch. Virol. 147: 1189-1195 discloses a mouse model of human CMV inherent CNS infection to study human CMV congenital infection in the CNS and to provide basic research for preparing a human CMV vaccine.

### SUMMARY OF THE INVENTION

A new transmission route of human herpesvirus has been found that allows developing an animal model that is useful for identifying potentially therapeutic compounds, particularly compounds that are potentially useful in treating and/or preventing infections and diseases caused by human herpesviruses. In particular, an animal model that is useful for testing compounds for the purpose of identifying potentially prophylactic or therapeutic compounds has been developed based on the new vertical mother-progeny transmission route. This model, which comprises experimentally infecting a non-human female animal with human herpesviruses before or after the administration of the compound to be tested, crossbreeding her with a male of the same species and analyzing the presence of human herpesviruses in the progeny and/or determining the effect of said compound on the progeny or the mother (non-human female animal infected with a human herpesvirus). Instead of the mother, the compounds to be tested can alternatively be administered to the descendants carrying human herpesviruses and the effect on such animals can be analyzed.

A method such as the one provided by this invention allows (i) identifying compounds that are potentially useful for preventing the vertical transmission of the virus from the mother to the progeny, which could be used as vaccines or as antiviral agents, as well as (ii) evaluating compounds that are potentially useful for treating or preventing infections and diseases caused by human herpesviruses and/or for treating the clinical symptoms associated with such diseases, which could be used, for example as antiviral, neuroprotective, anti-neurodegenerative agents, etc.

The animal model developed in this invention, based on the discovery of the vertical mother-progeny transmission of the human herpesvirus, allows consistently and reproducibly replicating the infection caused by human herpesviruses, particularly the neurotropism of some human herpesviruses, so it can be used to test compounds that are potentially therapeutic for treating and/or preventing infections and diseases caused by human herpesviruses.

Therefore, in one aspect, the invention relates to a method for identifying a potentially therapeutic compound which comprises experimentally infecting a non-human female animal with human herpesviruses before or after administering the compound to be tested, crossbreeding her with a male from her species and analyzing the presence of human herpesviruses in the progeny and/or determining their effect on the progeny.

An alternative method for identifying a potentially therapeutic compound comprises experimentally infecting a non-human female animal with human herpesviruses, crossbreeding her with a male from her species, selecting the descendants carrying human herpesviruses, administering the compound to be tested to said descendants and determining the effect caused by the compound to be tested on said descendants carrying human herpesviruses.

In another aspect, the invention relates to a method for producing a non-human animal carrying human herpesviruses which comprises infecting a non-human female animal with a human herpesvirus, crossbreeding her with a male from her species and selecting the descendants carrying human herpesviruses, which constitute an additional aspect of this invention.

### BRIEF DESCRIPTION OF THE FIGURES

Figure 1 is a Kaplan-Meier graph showing the percentage of descendant mice (from neonates to adults) of mothers infected with HSV-1 that survived post-delivery and their comparison with the descendants of MOCK mothers.
Figure 2 is a bar diagram showing the results, according to sex, of the mortality of the neonates (days 1 or 2 post-delivery) of mothers infected with HSV-1.
Figure 3 is a bar diagram showing viral loads in the spinal cord, encephalon and placenta of the embryos (day before birth) of mothers infected with HSV- 1 and the percentages of infected embryos.
Figure 4 is a bar diagram showing viral loads in the blood and encephalon of neonates of mothers infected with HSV-1 and the percentages of infected neonates
Figure 5 is a bar diagram showing viral loads in the brain, spinal cord, blood, gonads and trigeminal ganglia of 14-week old male and female adults born to infected mothers.
Figure 6 is a bar diagram showing the viremia of the females pre-delivery, neonates, mothers post-delivery and the sum of neonates and mothers after birth.
Figure 7 is a bar diagram illustrating the effect of acyclovir on the mothers (Figure 7A), particularly the viral loads in the blood, brain and trigeminal ganglia, as well as the effect of acyclovir on the progeny (Figure 7B), particularly viral loads in the blood, brain and spinal cord.

### DETAILED DESCRIPTION OF THE INVENTION

The meaning of some terms and expressions used in the context of the invention are described below in order to aid in understanding the invention object of this patent application.

The expression "non-human animal" refers to any non-human animal species susceptible of being infected by a human herpesvirus, both the wild type (wt) and the genetically manipulated type, for example genetically manipulated so as to incorporate a mutation (deletion, insertion or alteration) giving rise to a modification of the "wt" genotype or phenotype, for example non-human animals that are transgenic or mutant/deficient in a gene (KO), such as for example the APP gene, APOE gene, etc. Said non-human animal can be, for example, a fish, a non-human mammal, such as a rodent, a primate, a suid, etc., preferably a rodent, for example a mouse, a rat, a guinea pig, etc.; in a particular embodiment, the non-human animal used and susceptible of being infected by a human herpesvirus is a female mouse or fish.

The term "human herpesvirus" includes any human herpesvirus, for example HSV-1, HSV-2, VZV, CMV, EBV, HHV-6, HHV-7 or HHV-8, and mixtures thereof; said term includes both wild type (wt) virus and genetically manipulated viruses; by way of illustration, said viruses are human herpesviruses that are genetically manipulated to eliminate some of their genes or to incorporate a marker, for example β-galactosidase, luciferase, alkaline phosphatase, fluorescent marking proteins such as green fluorescent protein (GFP), cyan fluorescent protein (CFP), red fluorescent protein (RFP), yellow fluorescent protein (YFP), etc.; the present invention can be carried out by experimentally infecting with a human herpesvirus or with a mixture of two or more different human herpesviruses.

The term "potentially therapeutic compound" refers to a compound that is able to have an effect on the non-human female animal experimentally infected with a human herpesvirus or on her progeny (descendants), as well as an effect on the viral load, for example preventing the vertical mother-progeny transmission of human herpesviruses and/or reducing or eliminating the human herpesvirus load in said experimentally infected female or in her progeny or in successive generations, or its consequences on, for example, their viability, their behavior, their possible modifications in organs of interest, their possible cytogenetic or neuropathological alterations.

Said compound can be a compound of any nature, for example a chemical, biological, microbiological compound, etc., isolated or mixed with one or more different compounds, and it includes compounds of a known or unknown composition and structure, pharmaceutical products with a known therapeutic application, biological products, microbiological products, etc., for example organic or inorganic chemical compounds, peptides, proteins, nucleic acids, extracts, etc..

The viral load can be determined by any conventional method, such as any of the methods defined in relation to step d) of the method of the invention (see below). By way of illustration, in order for a compound to be considered "potentially therapeutic" said compound has to (i) prevent the vertical mother-progeny transmission of human herpesviruses, which can easily be evaluated by determining the presence or absence of human herpesviruses in the progeny of a female experimentally infected with human herpesviruses, (ii) reducing the viral load of the experimentally infected female or in a descendant of her progeny carrying human herpesviruses by at least 5% with respect to the viral load of the mother or her descendant immediately before the administration of the compound to be tested, for example at least 10%, or at least 20%, or at least 30%, or at least 40%, or at least 50%, or at least 60%, or at least 70%, or at least 80%, or at least 90%, or at least 95% with respect to the viral load of the mother or her descendant immediately before administration of the compound to be tested; and/or (iii) reversing, reducing or minimizing the consequences of human herpesviruses in the experimentally infected female or in her progeny or in successive generations, for example on their viability, their behavior, their possible modifications in organs of interest, their possible cytogenetic or neuropathological alterations.

The present invention is based on the discovery that human herpesviruses can be vertically transmitted from mothers to progeny. Therefore by analyzing the progeny of a mother experimentally infected with human herpesviruses that was administered before or after said experimental infection a potentially therapeutic compound, or the mother herself, it is possible to evaluate the possible therapeutic use of said compound in preventing and/or treating infections and diseases caused by human herpesviruses. Also, by administering a potentially therapeutic compound to the descendants of a mother experimentally infected with human herpesviruses that was not previously administered said compound to be tested, it is possible to evaluate the possible therapeutic use of the tested compound in preventing and/or treating infections and diseases caused by human herpesviruses and its consequences.

Therefore, in one aspect, the invention relates to a method for identifying a potentially therapeutic compound, hereinafter, method of the invention, comprising the steps of:
a) infecting a non-human female animal with a human herpesvirus;
b) administering the potentially therapeutic compound to be tested to said non-human female animal;
c) crossbreeding said non-human female animal infected with a human herpesvirus with a non-human male animal belonging to the same species as said female, and
d) analyzing the presence of human herpesviruses in the progeny of said non-human female animal infected with a human herpesvirus and/or determining the effect of said potentially therapeutic compound on said progeny,
in which steps a) and b) are performed in any order.

In a particular embodiment, the method of the invention comprises performing step a) [experimentally infecting the non-human female animal with a human herpesvirus] before step b) [administering the non-human female animal the potentially therapeutic compound to be tested]. This particular embodiment of the method of the invention is hereinafter referred to as Method A.

In another particular embodiment, the method of the invention comprises performing step b) [administering the potentially therapeutic compound to be tested to the non-human female animal] before step a) [experimental infection of the non-human female animal with a human herpesvirus]. This particular embodiment of the method of the invention is hereinafter referred to as Method B.

Method A begins with experimentally infecting the non-human female animal with a human herpesvirus [step a)]. Said infection can be carried out using any of the known human herpesvirus infection routes, for example the olfactory, neural or hematogenous route. In a particular embodiment, the infection is carried out following the hematogenous route, administering human herpesvirus to the female by means of intraperitoneal (i.p.) and/or intravenous (i.v.) injection (for example by puncturing a vein), advantageously by means of i.p. injection or 2 injections (i.p. + i.v.). Infection through the olfactory route can be carried out by means of intranasal administrations, whereas infection through the neural route can be carried out by different methods, for example by causing bilateral wounds by abrading the non-human animal's nose with a blade and applying solutions with the virus, which mimics cold fevers occurring in humans. The human herpesvirus load to be administered to the female to be experimentally infected can vary within a wide range; nevertheless, in a particular embodiment, said viral load is comprised between 1 and 10⁶ plaque forming units (pfu). Despite the viral specificity and the limited range of human herpesvirus hosts that could prevent the effective infection of species that are not the natural hosts of said viruses, good infection rates are infection rates are obtained when non-human animals are infected with human herpesviruses according to the methodology described in the present invention.

Then the potentially therapeutic compound to be tested is administered to the non-human female animal infected with human herpesvirus [step b)]. Said compound is administered to the previously infected female by any suitable administration route (for example orally, subcutaneously, parenterally, for example i.v. or i.p., etc.), in an administration form that is suitable for the chosen administration route and at a suitable dosage; by way of illustration said potentially therapeutic compound can be administered in the form of a single dose or step or in several doses or steps over time, or by means of a continuous supply of the compound to be tested.

Then the female infected with human herpesvirus that was administered the compound to be tested is crossbred [step c)], by conventional methods known by persons skilled in the art, with a non-human male animal belonging to the same species as said female. The male that the infected female is crossbred with may or may not be free of human herpesviruses; in principle this fact seems to be irrelevant since for transmission it would not matter if the father were infected or were MOCK (that is, free of human herpesviruses), provided that this did not affect the ability of impregnating the female, which does not seem to occur; nevertheless, in a particular embodiment MOCK males are used in the experimental design. Finally the presence of human herpesviruses in the progeny of said non-human female animal infected with a human herpesvirus is analyzed and/or the effect of said potentially therapeutic compound on said progeny is determined [step d)].

In a particular embodiment, the presence of human herpesviruses in the progeny of the experimentally infected female is analyzed. Said analysis can be carried out by any suitable method that allows detecting the presence of human herpesviruses in the tested sample. Such methods include serological (immunological) or genetic methods, etc., and the use of suitable molecular marker detection systems based, for example, on fluorescence, luminescence, colorimetric reactions, etc. By way of illustration, ELISA or Western-blot protein detection assays, methods based on the use of bioluminiscent, fluorescent viruses, etc., methods based on the immunocytological and immunohistological detection of human herpesviruses, cell monolayer plating assays, viral-based DNA identification methods, for example methods based on the use of oligonucleotide probes marked with detectable markers, or based on carrying out carrying out specific enzymatic reactions such as restriction reactions or amplification reactions, based for example on polymerase chain reaction (PCR), etc., can be used. In a particular embodiment, the possible presence of human herpesviruses in progeny is carried out in a sample from the animals of the progeny by means of real-time quantitative PCR according to a previously defined protocol [Burgos JS, Ramirez C, Sastre I, Bullido MJ and Valdivieso F. (2003). ApoE4 is more efficient than E3 in brain access by herpes simplex virus type 1. NeuroReport 14(14); 1825-1827; Burgos JS, Ramirez C, Sastre I, Bullido MJ and Valdivieso F. (2002). Involvement of apolipoprotein E in the hematogenous route of herpes simplex virus type 1 to the central nervous system. Journal of Virology. 76(23); 12394-12398; Burgos JS, Ramirez C, Tenorio R, Sastre 1 and Bullido MJ. (2002). Influence of reagents formulation on real-time PCR parameters. Molecular and Cellular Probes. 16; 257-260].

In another particular embodiment the effect of the tested compound on the progeny of the experimentally infected female is determined. The presence of human herpesviruses can thus be determined in the progeny, as well as the pathological changes associated with the presence of viruses in the progeny. The effect of the tested compound on said progeny can be determined by means of different conventional experimental techniques based, for example, on image analysis (for example nuclear magnetic resonance, bioluminescence, computerized tomography, positron emission tomography or PET, fluorescence, etc.), histopathological techniques, detection of viral and neurodegeneration markers both at the genomic and proteomic levels, etc.

By way of illustration, the effect of the tested compound on the female experimentally infected with human herpesviruses or on her descendants can be determined by means of performing at least one analysis or study selected from (1) viability analysis of the female progenitors and of the progeny, (2) behavior analysis of the female progenitors and of the progeny, (3) image studies of the female progenitors and of the progeny; (4) cytogenetic analyses of the progeny, and (5) neuropathological studies.
(1) Viability analysis of female progenitors and descendants: Survival rates are determined by means of this analysis both in the progeny and in the female progenitor and it evaluates if the tested compound modifies the viability percentage in the animals administered the compound with respect to the controls.
(2) Behavior analysis of the female progenitors and descendants: The complete study of diseases in animal models includes a behavior analysis of the animal. This study is useful for checking the effect of the viral infection on brain function both in the descendants and in the female progenitor. In a particular embodiment, the animal model is a murine model, in which case the behavior analysis of said murine model for a disease can be subdivided into three experimental phases: (i) phase of evaluating their state of health and neurological reflexes; (ii) phase of analyzing their motor and sensory ability; and (iii) model-specific phase with specific tests validated for the specific neuropathology. Generally, the animal's weight, the condition of their coat, reaction to being handled by the investigator, auditory reflexes, visual reflexes, olfactory capability and social behaviors are checked in the first phase [(i)]. The second phase [(ii)] consists of a group of tests for checking if the animal's condition is suitable when performing the specific subsequent tests. This group of tests includes studying the auditory capability, visual capability, motor functions or the coordination of movements, among others. Tests specific to the pathology under study are carried out in the third phase [(iii)], including those described in the literature, with the fundamental characteristic that they are standardized tests that can be validated and are reproducible. By way of illustration, the test that is most used in Alzheimer's disease animal models for checking the mouse's memory is the Morris tank. This test analyzes the mouse's ability to remember the location of a platform submerged in a bath, exclusively using a series of visual clues located outside the tank. For Parkinson's disease animal models, the learning capability is studied in the *Rotarod.* This test can analyze the motor learning of a mouse. The mouse must adapt to the accelerated movement of a cylinder, in which motor coordination and learning thereof are evaluated.
(3) Image studies in female progenitors and descendants: Image techniques for small animals, which are non-invasive and allow performing the different studies without sacrificing the animal, can be applied for the purpose of studying modifications at the cerebral level and in other organs of interest. Included among the techniques most used for this purpose are nuclear magnetic resonance (NMR), positron emission tomography (PET), computer-assisted tomography (CAT), high-resolution X-rays and bioluminescence imaging (BLI) based on enzymatic reactions and on methods of implanting genetic constructs with fluorescent proteins, for example, GFP. The various image analysis techniques can be used to detect morphological and pathological changes *in vivo,* and for monitoring the human herpesvirus in the female progenitors and progeny.
(4) Cytogenetic analyses in the progeny: Chromosomal aberrations, structural genetic changes and variation of the number of copies in the progeny of experimentally infected mothers, studying the genetic consequences of this fact, can be studied for this purpose. To that end, cytogenetic maps of the descendants as well as strategies for searching for structural modifications by fluorescent *in situ* hybridization [FISH] are carried out. Chromosomal structural variations to be studied may be: deletions, duplications, inversions and/or translocations. Chromosomal numerical variations to be studied include: polyploids, haploids and/or aneuploids. If desired, the possible transmission of said chromosomal variations may also be studied.
(5) Neuropathological studies in female progenitors and in descendants: The regions of the brain showing signs of degeneration in the female progenitors and in their descendants can be determined by means of histopathological techniques. Furthermore, some neurodegeneration neuronal markers, such as α-synuclein, APP (amyloid precursor protein), ApoE (apolipoprotein E), tau phosphorylation state, etc., can be studied by means of immunohistochemical techniques. These antigens of interest can be detected together with specific neuronal-type markers, such as tyrosine hydroxylase, which marks dopaminergic neurons, or acetylcholine transferase, a cholinergic neuronal marker, for the purpose of determining the loss of specific neuron populations. In this case, the protein markers involved in neurodegenerative processes would be analyzed under different infection conditions, comparing them with variations in endogenous proteins. The mRNA of the cell and viral genes involved in neuroinvasion and neurodegeneration can also be analyzed as a supplement to the immunohistochemical studies by means of quantitative analysis with RT-PCR of the corresponding messengers, as well as the quantitative variability of the total protein levels of crude extracts by means of the Western-blot technique.

The results obtained in Examples 1 to 3 enclosed in this description show: (i) vertical mother-progeny transmission of human herpesviruses and the location of the virus mainly in the descendants' blood and brain; (ii) the administration of acyclovir to the mothers reduces mortality levels observed in the descendants of the untreated animal; (iii) the viral load levels in the different organs analyzed both in the mother and progeny decrease when the mothers are treated with acyclovir; and, (iv) the oral administration of acyclovir is more efficient than the subcutaneous administration.

Therefore, the invention shows the existence of vertical transmission of human herpesviruses from experimentally infected mothers to progeny. Although the inventors do not wish to be linked to any hypothesis, it is believed that this mother-progeny transmission mainly occurs by means of the hematogenous route since the larger viral load in the neonate is found in the blood it shares with the mother. According to this hypothesis, the mother would be supplying the virus to the progeny during the entire gestation through blood. Therefore, the presence of the human herpesvirus in progeny would be indicative that the compound tested in the mother is not able to eliminate the herpesvirus in the mother and, therefore, it occurs in her descendants. In contrast, the absence of human herpesvirus in the progeny would be indicative that the compound tested in the mother is able to eliminate the herpesvirus in the mother or to prevent the vertical mother-progeny transmission, and, therefore can act as an antiviral agent.

Method A, comprising the administration the compound to be tested to the previously infected mother allows identifying potentially useful compounds because they have an effect on the mother or on the progeny, as a result of the presence of human herpesviruses, at the level of survival, behavior, modifications in organs of interest and/or neuropathologies or as antiviral agents (the absence or reduction in the viral load of the experimentally infected mother illustrates that the administered compound is potentially useful as an antiviral agent).

Method B begins with the administration of the potentially therapeutic compound to be tested to the non-human female animal [step b)], and then said female pretreated with a human herpesviruses is experimentally infected [step a)]. These steps are carried out in a manner similar to that in which steps a) and b) defined in relation to Method A are carried out. Steps c) and d) of Method B are the same as those of Method A and are carried out in the same manner.

Method B, comprising the administration to the female of the compound to be tested before she is experimentally infected allows identifying potentially therapeutic compounds that are useful as vaccines or as agents that are capable of preventing the mother-progeny transmission of the virus. To identify the compounds that are potentially useful as vaccines, the target females of the experimental infection must be females with no signs of human herpesvirus infection; advantageously females that are free of such viruses (MOCK), for this reason such females are previously analyzed by means of any conventional method that allows detecting the presence of human herpesviruses and determining whether or not they were in contact with such viruses, by means of any of the previously mentioned methods. The absence of human herpesviruses in the progeny of the experimentally infected female illustrates that the potentially therapeutic compound administered to the mother is potentially useful as a vaccine or as an agent that is able to prevent vertical mother-progeny transmission of the virus. Said Method B likewise allows identifying compounds exercising an effect on the mother or on the progeny at the level of survival, behavior, modifications of organs of interest and/or neuropathologies as a consequence of the presence of human herpesviruses.

An alternative method for identifying a potentially therapeutic compound, hereinafter Method C, comprises the steps of:
i) infecting a non-human female animal with a human herpesvirus;
ii) crossbreeding said non-human female animal infected with a human herpesvirus with a non-human male animal belonging to the same species as said female;
iii) analyzing the presence of human herpesviruses in the progeny of said non-human female animal infected with a human herpesvirus and selecting those descendants carrying human herpesviruses;
iv) administering the potentially therapeutic compound to be tested to said descendants carrying human herpesviruses; and
v) determining the effect of said potentially therapeutic compound tested on said descendants carrying human herpesviruses.

Method C begins with the experimental infection of the non-human female animal with a human herpesvirus [step i)]. Said experimental infection is carried out in a manner similar to that in which it is carried out in step a) of Method A. Then, the female infected with human herpesvirus is crossbred [step ii)] with a non-human male animal belonging to the same species as said female in a manner similar to how it is carried out in step c) of Method A. Then, the presence of human herpesviruses in the progeny of the non-human female animal infected with human herpesvirus is analyzed and those descendants carrying human herpesviruses are selected [step iii)]. The presence of human herpesvirus in the progeny of the experimentally infected female is analyzed by any suitable method in a manner similar to that indicated in relation to step d) of Method A. Then the descendants carrying human herpesviruses are administered the potentially therapeutic compound to be tested in the form of a single dose or step or in several doses or steps over time or by means of a continuous supply of the compound to be tested in a manner similar to that indicated in relation to step b) of Method A and, finally the effect of said potentially therapeutic compound tested on said descendants carrying human herpesviruses is determined. Said effect may consist of the total or partial reduction of the viral load of the animal carrying human herpesviruses, in which case said potentially therapeutic compound is a said potentially therapeutic compound is a compound that is potentially useful as an antiviral agent, or of an effect related to the consequences of the presence of human herpesviruses in said animal at the level of survival, behavior, modifications of organs of interest and/or neuropathologies, to which end at least an analysis or study on said descendants carrying human herpesviruses can be carried out, selected from (1) viability analysis, (2) behavior analysis, (3) image studies, (4) cytogenetic analyses, and (5) neuropathological studies.

Since human herpesviruses are related to various human diseases, for example labial or genital herpes, varicella, infectious mononucleosis, nasopharyngeal carcinoma, pneumonia, retinitis, sudden exanthema, Kaposi's sarcoma, CNS infections, neurological diseases and neuropathological disorders such as encephalitis; Alzheimer's disease, boxer's dementia, HIV-associated dementia, cerebral paralysis, meningitis, meningoencephalitis and myelitis, said potentially therapeutic compound can be used for the treatment or prophylaxis of such pathologies insofar as they are caused by human herpesvirus. Therefore, in a particular embodiment, the potentially therapeutic compound susceptible ofbeing identified by means of any of the previously defined Methods A, B and C, is a vaccine, an antiviral agent, a neuroprotective agent or an anti-neurodegenerative agent.

In another aspect, the invention relates to a method for producing a non-human animal carrying human herpesviruses, comprising the steps of:
a) infecting a non-human female animal with a human herpesvirus;
b) crossbreeding said non-human female animal infected with a human herpesvirus with a non-human male animal belonging to the same species as said female, and
c) selecting the descendants carrying human herpesviruses.

Said method begins with the experimental infection of the non-human female animal with a human herpesvirus [step a)], which is carried out in a manner similar to that in which it is carried out in step a) of Method A; then, the female infected with human herpesvirus is crossbred [step b)] with a non-human male animal belonging to the same species as said female in a manner similar to that in which it is carried out in step c) of Method A; and finally the presence of human herpesviruses in the progeny of the non-human female animal infected with human herpesvirus is analyzed and those descendants carrying human herpesviruses [step c)] are selected. The presence of human herpesvirus in the progeny of the experimentally infected female is analyzed by any suitable method in a manner similar to that indicated in relation to step d) of Method A.

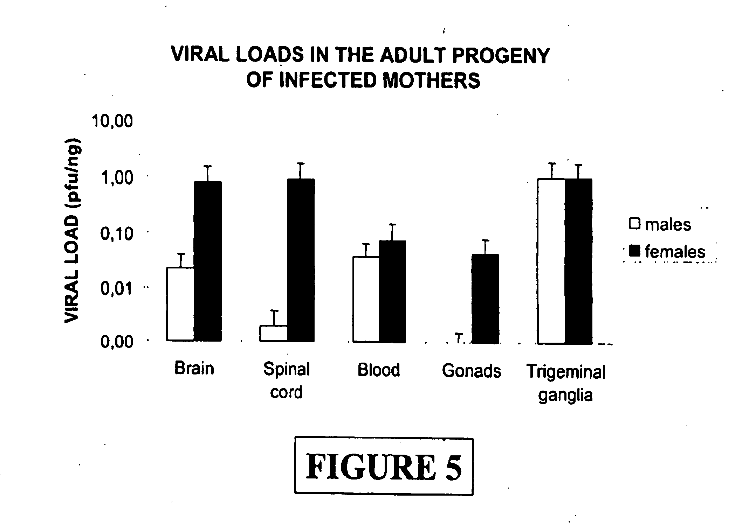

Descendants carrying human herpesviruses can be obtained. Non-human animals carrying human herpesviruses can be obtained according to the previously described method. The non-human animal carrying human herpesviruses is characterized by being a descendant of a non-human animal infected with a human herpesvirus. Additionally, said non-human animal has the characteristic that said human herpesviruses are mainly located in the central nervous system (CNS) and/or in blood, for example in the brain, spinal cord and/or blood. As shown in Example 1 and in Figure 5, it was again observed in 14-week old progeny of infected mothers that the target organs for HSV-1 infection by vertical transmission are CNS and blood, corroborating prior results. Furthermore, both in males and females, the trigeminal ganglia showed high levels of the virus. One of the major differences between sexes was the colonization of the gonads, HSV-1 being undetectable in testicles. In a particular embodiment, the non-human animal carrying human herpesviruses provided by this invention is a fish, a rodent, a primate or a suid, for example a mouse, a rat, a guinea pig, a monkey or a pig, either male or female. These animals can be used both as animal models for experimenting and searching for potentially therapeutic compounds and as progenitors for new descendants carrying human herpesviruses.

Therefore, in another aspect the invention relates to an alternative method for producing a non-human animal carrying human herpesviruses, comprising the steps of:
a) crossbreeding a first non-human animal carrying human herpesviruses descending from a non-human female animal infected with a human herpesvirus with a second non-human animal that belongs to the same species as said first animal but of a different sex, and
b) selecting the descendants carrying human herpesviruses.

Said method comprises crossbreeding a first non-human animal carrying human herpesviruses that is a descendant of a non-human female-animal infected with a human herpesvirus with a second non-human animal belonging to the same species but of a different sex. This second animal can be a MOCK animal or, alternatively a non-human animal carrying human herpesviruses provided by this invention that is a descendant of a non-human female animal infected with a human herpesvirus. The crossbreeding of said animals is carried out in a conventional manner, as previously mentioned. Then the presence of human herpesviruses in the descendants is analyzed and the descendants carrying human herpesviruses are selected, which can be used as animal models for reproductive purposes for crossbreeding new non-human animals carrying human herpesviruses.

The following Examples are useful for illustrating the invention and must not be considered to be limiting thereof.

### EXAMPLE 1

### Vertical mother-progeny transmission of human herpesviruses

All the animals included in the experimental process were 14-week old female mice from the C57B1/6 lineage: Sixteen adult mice (females) were used as progenitors, and 21 embryos, 77 neonates and 13 adult progeny were used. The experiments were carried out strictly following the Guidance on the Operation of Animals (Scientific Procedures, Act 1986), being supervised by personnel of the Animal house of the Centro de Biologia Molecular (*Molecular Biology Center)* Severo Ochoa. All the animals went through a quarantine period and were treated with strict precautions against contamination during inoculation and dissection.

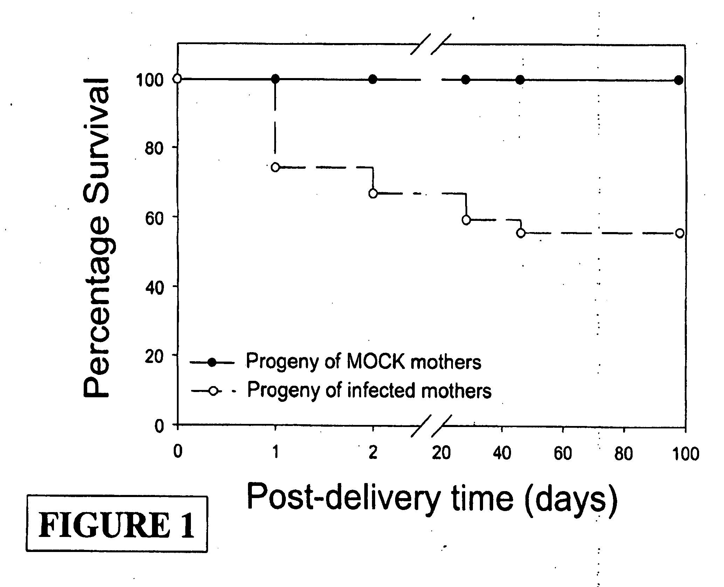

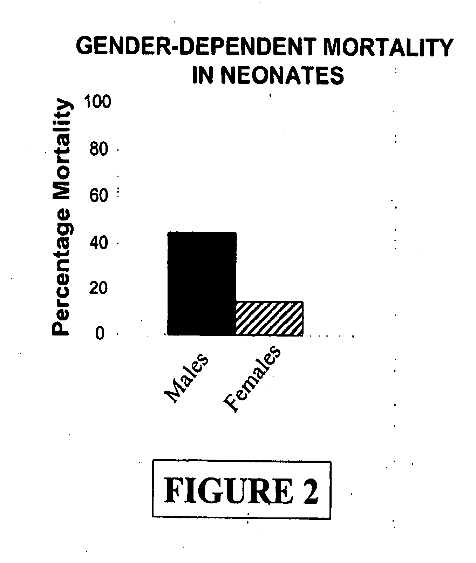

The animals were intraperitoneally infected with a dose of 10⁶ plaque forming units (pfu) of herpes simplex virus type 1 (HSV-1) of the KOS strain (provided by Dr. L. Carrasco, Centro de Biologia Molecular Severo Ochoa, Universidad Autónoma of Madrid). After 37 days post-infection, at which time latent infection was already established (latency is considered after day 28 post-infection), the females were crossbred with male mice and mortality of the descendants between days 1 and 100 post-delivery was analyzed. A comparison was carried out by means of a Kaplan-Meier graph between the descendants of infected mothers and the descendants of MOCK mothers. The results are shown in Figure 1 and illustrate a reduction in the survival of the descendants of infected mothers. The sex-dependent mortality in the vertical transmission of HSV-1 in neonates at days 1 or 2 post-delivery was additionally analyzed, observing that the males have a higher percentage of neonate mortality (45%) than females (15%), as shown in Figure 2.

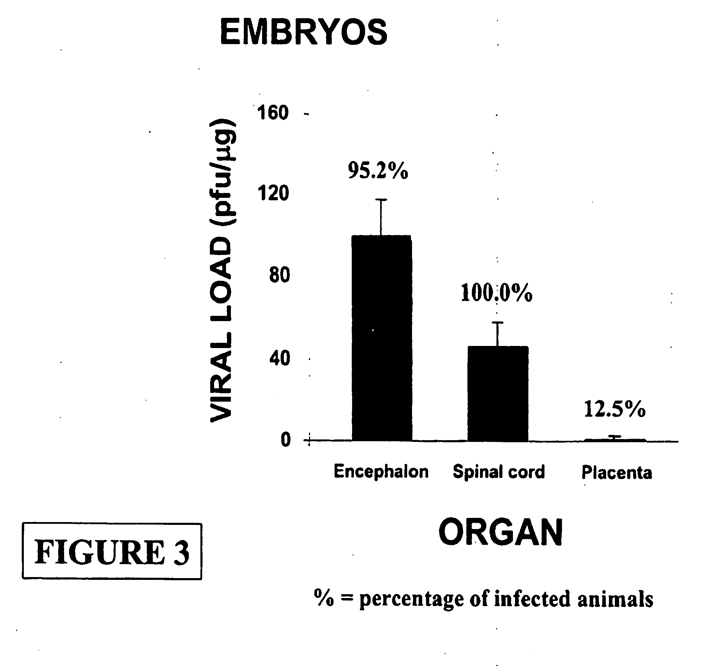

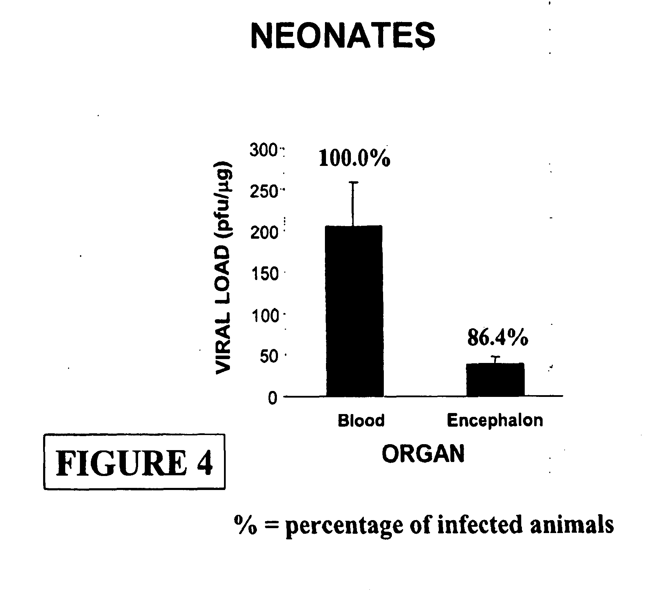

On the other hand, the fetuses were extracted and their organs were dissected and frozen the day before birth. Then, the HSV-1 DNA was extracted and quantified in the different analyzed organs (encephalon, spinal cord, placenta) by means of quantitative real-time PCR (expressed as equivalent pfu) and normalized with respect to the mouse actin gene (expressed as (expressed as ng). The DNA was extracted using conventional methods (NucleoSpin^{®}, Cat. K3053-2, ClonTech, USA). The cross-contamination of samples and the PCR false positives were carefully prevented by frequently changing gloves, exclusive use of pipettes and strictly separating the three main PCR steps. Quantitative PCR was carried out using the LightCycler thermal cycler (Roche Diagnostics Ltd, Lewes, UK), the reaction mixture of which contained I µM of primers and 2 mM of MgCl₂. The β-actin primers (SEQ. ID. NO: 1 and SEQ. ID. NO: 2) were used as positive control for the PCR reaction (in order to obtain a 379 base pair [bp] amplicon or amplification product). Primers specific for amplifying a 120 bp fragment of the sequence of the viral DNA polymerase (pol) gene [SEQ. ID. NO: 3 and SEQ. ID. NO: 4] were further used, as well as primers specific for amplifying a 110 bp fragment of bases of the sequence of the viral thymidine kinase (TK) gene [SEQ. ID. NO: 5 and SEQ. ID. NO: 6]. The PCR conditions were one cycle of 95°C for 1 minute, followed by 45 cycles at 95°C for 30 seconds; 55°C (for β-actin and TK) or 60°C (for pol) for 30 seconds; and 72°C for 40 seconds. The virus concentration interval for optimizing the quantitative real-time PCR standard curve was expressed as pfu. To calibrate the β-actin gene, nanograms (ng) were used as the unit of this endogenous gene. The identity of the amplified products was checked by analysis of the denaturation curves, gel electrophoresis and restriction analysis. The gene fragments analyzed were subjected to a restriction analysis with the endonuclease Aval for viral polymerase (pol) (producing two 23 and 97 bp fragments) and for viral TK (producing two 35 and 75 bp fragments) and with the enzyme N1a1V for β-actin (producing two 220 and 159 bp fragments). The viral DNA detection results in embryos are shown in Figure 3, whereas the viral loads in the neonates is shown in Figure 4.

The viral load results in adults descending from infected mothers separated by sex are shown in Figure 5. The results correspond to 5 males and 8 females. As can be observed in said Figure 5, it was again observed in the 14-week old progeny of infected mothers that the target organs for HSV-1 infection by vertical transmission are the CNS and blood, corroborating earlier results. Furthermore, both in males and in females, the trigeminal ganglia showed high virus levels. One of the major differences between sexes was colonization of the gonads, HSV-1 being undetectable in testicles.

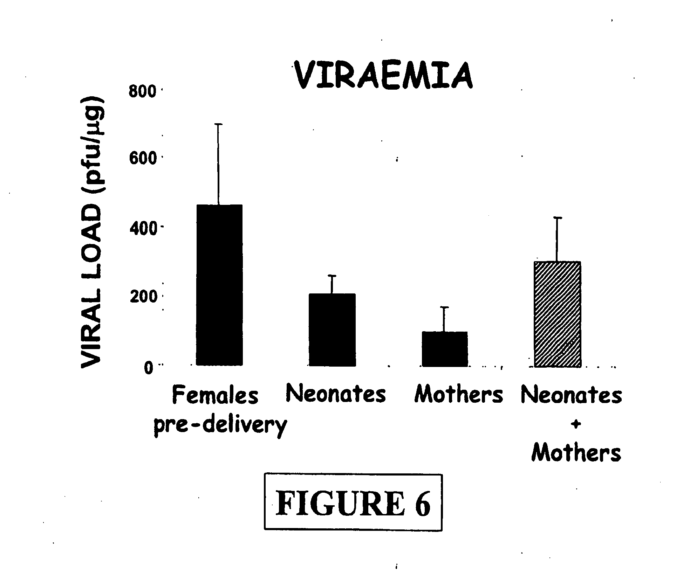

The previously described murine model was used to evaluate the effectiveness of the vertical hematogenous transmission of HSV-1 from the mother to the descendants. Viremia in pre-delivery females, neonates (1 or 2 days after birth) and in post-delivery mothers as well as the sum of the latter two categories is represented in Figure 6. The graph indicates that transmission is dependent on viremia and is useful for analyzing vaccines and antiviral agents by means of a hematogenous administration route.

### EXAMPLE 2

### Effect of an antiviral agent on vertical mother-progeny transmission of human herpesviruses

Example 1 shows the existence of vertical mother-progeny transmission of HSV-1. These results indicate that HSV-1 was found both in blood and in the brain of the descendants on day 1 post delivery, and in the CNS of embryos on day-1 before birth. This group of results implies that the interruption of the vertical transmission of HSV-1 could have potential therapeutic properties (vaccine, antiviral or neuroprotective properties) on the descendants, resulting from the elimination of HSV-1 in the progeny's CNS. Based on said hypothesis, the inventors decided to evaluate the antiviral role of the classic anti-herpes product of choice, acyclovir, which has been proven to be non-teratogenic or embryotoxic in rabbits, rats or mice. To that end, and after establishing a latent infection in adult females, treatment with acyclovir, crossbreeding and analysis of the mothers and progeny were carried out.

### Materials and methods

All the animals included in the experimental process were 14-week old female mice of the C57Bl/6 lineage. Between two and three adult mice were used per analysis group and between 13 and 25 neonates, depending on the availability of animals. The experiments were performed strictly following the Guidance on the Operation of Animals (Scientific Procedures, Act 1986), supervised by personnel of the Animal house of the Centro de Biologia Molecular Severo Ochoa. All the animals went through a quarantine period and were treated with strict precautions against contamination during inoculation and dissection. The animals were intraperitoneally infected with a dose 10⁶ pfu of herpes simplex virus type 1 (HSV-1) of the KOS strain (provided by el Dr. L. Carrasco, Centro de Biologia Molecular Severo Ochoa, Universidad Autónoma of Madrid). After 37 days post-infection, at which time latent infection was already established, the mice were separated into three different basins corresponding to the control (untreated), oral acyclovir and subcutaneous acyclovir (administration of which is slower) groups, and treatment with acyclovir began five days before crossbreeding until birth, crossbreeding the animals at day 42 post-infection. The first group of mice was not given any treatment. The oral acyclovir group was administered 100 µl of a 2.5 mg/ml dilution of the drug three times a day with intervals of at least 8 hours of difference (since the plasma half-life of acyclovir after administration is 2.9 h). Another group of animals was injected 100 µl of subcutaneous acyclovir at a dilution of 2.5 mg/ml three times a day (every 8 hours) coinciding with the previous group. Therefore the antiviral concentration administered per dose and mouse was 0.25 mg (10 mg/kg per mouse, far from the toxicity limit of 80 mg/kg cited above), with a total daily dose of 0.75 mg/day. The acyclovir administration process concluded on the day of birth, at which time euthanasia was performed on the mothers and the respective progeny for subsequent analysis.

The dissected organs in the adult animals were the following: blood, ovaries, adrenal gland, spinal cord, brain, cerebellum and trigeminal ganglia. The entire brain was separated into three rough regions: midbrain, ventricles and cerebral cortex. In the neonates, the dissected organs were blood, spinal cord and encephalon. DNA was extracted using conventional methods (NucleoSpin^{®}, Cat. K3053-2, ClonTech, USA). The viral load of the different organs was analyzed by means of quantitative PCR. Cross-contamination of samples and PCR false positives were carefully prevented by frequently changing gloves, exclusive use of pipettes and strictly separating the three main PCR steps. Quantitative PCR was performed using the LightCycler thermal cycler (Roche Diagnostics Ltd, Lewes, UK), the reaction mixture of which contained 1 µM of primers and 2 mM of MgCl₂. β-actin primers (SEQ. ID. NO: 1 and SEQ. ID. NO: 2) were used as positive control for the PCR reaction (obtaining a 379 bp product). Primers specific for the sequence of the viral polymerase (pol) gene were used to detect the virus (SEQ. ID. NO: 3 and SEQ. ID. NO: 4) (120 bp amplicon). The PCR conditions were one cycle of 95°C for 10 minutes, followed by 45 cycles at 95°C for 30 seconds, 55°C (for β-actin) or 60°C (for viral polymerase) for 30 seconds, and a last cycle of 72°C for 40 seconds. The virus concentration range used for optimizing the quantitative PCR standard curve was expressed as plaque forming units (pfu). For calibrating the β-actin gene, nanograms were used as the unit of this endogenous gene. The identity of the amplified products was checked by analysis of the denaturation curves, by gel electrophoresis and restriction analysis.

### Results and discussion

After birth and sacrifice, the progeny survival rates and the mother and neonate viral loads were analyzed. The adult animals remained asymptomatic during the entire experimental process, suffering no mortality. In contrast, the descendants of the various animal groups showed variable mortality rates. Therefore, the control group neonates showed mortality of 7.1% (1 *exitus* out of 14 animals), whereas the group corresponding to the administration of subcutaneous acyclovir showed a mortality rate of 4.0% (1 out of 25); finally, no mortality was observed in the group corresponding to the oral administration of the drug (none of the 17 animals). The number of females and males was not significantly different among the three groups of descendants (ratio of 1:1.66).

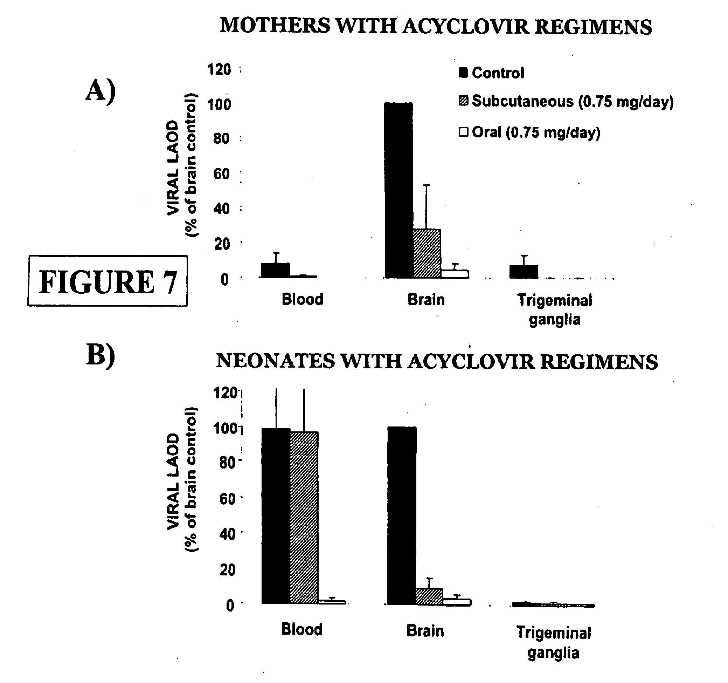

Regarding the virus levels in the various organs, and as can be seen in Figure 7, administration of acyclovir at a daily dose of 0.75 mg by means of the two administration routes evaluated produced a significant reduction of viral loads both in the mothers and in the progeny. The efficiency of the use of the subcutaneous administration of acyclovir was less than that of the oral administration both in mothers (Figure 7A) and in progeny (Figure 7B). Nevertheless, elimination of the virus in the animals with subcutaneous acyclovir was significant, observing considerably lower levels of the virus in all the analyzed organs of the mother and, specifically, in the progeny's CNS. The oral administration of acyclovir was still more effective in eliminating the virus in the mothers and, by extension, in the descendants. In this sense, virus levels in blood and in the trigeminal ganglia of the mothers were virtually undetectable, whereas in the brain of the orally treated animals, reduction of the virus level was 96%. Regarding the descendants, the oral administration of acyclovir in the mothers caused an almost complete elimination of viral levels in all the analyzed organs.

### Conclusions

The conclusions inferred from the trial performed are basically the following: (i) the vertical transmission phenomenon is confirmed in a new experiment, again observing that the virus is mainly located in the blood and brain of the descendant animals; (ii) it is shown that treating the mothers with acyclovir reduces the mortality levels observed in the descendants of the untreated animals; (iii) it is observed that the viral load levels in the various organs analyzed, both in the mothers and the progeny, decrease when the mothers are treated with acyclovir; and (iv), finally, it is observed that the administration of oral acyclovir is considerably more efficient than the subcutaneous administration of this antiviral drug.

The perspectives inferred from this trial indicate that not only acyclovir but also other antiviral agents, vaccines or neuroprotective agents could prevent the vertical transmission of HSV-1 and its subsequent effects on the CNS.

### EXAMPLE 3

### Effect of administration form of an antiviral agent on the vertical mother-progeny transmission of human herpesviruses

As the results observed in Examples 1 and 2 indicate that oral administration of acyclovir is more efficient than subcutaneous injection, an experiment of administering acyclovir in drink was carried out. For this experiment, 14 mice (5 controls and 9 treated with acyclovir) in which acyclovir was used *ad libitum* in the drink at 2 determined doses (4 animals with 250 mg/ml and 5 with 500 mg/ml) were used. In previous experiments, both oral and subcutaneous, 0.25 mg of acyclovir were administered per dose (100 µl of a dilution of 2.5 mg/ml), 3 times a day. Assuming that a mouse drinks about 3 ml a day, 2 working dilutions (250 mg/ml so that the final daily dose was 0.75 mg, and 500 mg/ml so that the final daily dose was 1.50 mg) were used. The administration of acyclovir began on the day the vaginal plug appears, at which time the mouse basin is changed to a new one with acyclovir. The obtained results show that survival of the animals treated with acyclovir that ingested acyclovir (high dose) with drinking water is higher.

## Claims

1. A method for identifying a potentially therapeutic compound, comprising the steps of:
a) infecting a non-human female animal with a human herpesvirus;
b) administering the potentially therapeutic compound to be tested to said non-human female animal;
c) crossbreeding said non-human female animal infected with a human herpesvirus with a non-human male animal belonging to the same species as said female, and
d) analyzing the presence of human herpesviruses in the progeny of said non-human female animal infected with a human herpesvirus and/or determining the effect of said potentially therapeutic compound on said progeny, wherein a compound is considered as potentially therapeutic if it results in a decrease of the viral load in the progeny and/or it is capable of decreasing or abrogating one or more of the symptoms associated with the infection by human herpesviruses wherein said symptoms are selected from (1) progeny behavior alteration, mainly reflexes, motor and sensory ability as well as disease specific symptoms, using behavior analysis, (2) structural modifications at cerebral level, (3) chromosomal aberrations, structural genetic changes and variation of the number of copies and (4) neurodegenerative processes,
wherein steps a) and b) are carried out in any order.

2. A method according to claim 1, wherein step a) is carried out before step b).

3. A method according to claim 1, wherein step b) is carried out before step a).

4. A method according to any of claims 1 to 3, wherein said non-human animal is a fish or a non-human mammal.

5. A method according to claim 4, wherein said non-human mammal is selected from a rodent, a primate and a suid.

6. A method according to claim 5, wherein said rodent is selected from a mouse, a rat and a guinea pig.

7. A method according to any of claims 1 to 3, wherein said human herpesvirus is selected from herpes simplex virus type 1 (HSV-1), herpes simplex virus type 2 (HSV-2), varicella-zoster virus (VZV), cytomegalovirus (CMV), human herpesvirus 6 (HHV-6), human herpesvirus 7 (HHV-7), Epstein-Barr virus (EBV) and Kaposi's herpesvirus (HHV-8), and mixtures thereof.

8. A method according to claim 1, wherein the determination of the effect of the tested compound on said progeny infected with a human herpesvirus comprises analyzing the viral load and/or performing at least one analysis or study selected from (1) behavior analysis, (2) image studies, (3) cytogenetic analyses, and (4) neuropathological studies.

9. A method for obtaining a non-human animal carrying human herpesviruses comprising the steps of:
a) infecting experimentally a non-human female animal with a human herpesvirus;
b) crossbreeding said non-human female animal infected with a human herpesvirus with a non-human male animal belonging to the same species as said female, and
c) selecting the descendants carrying human herpesviruses.

10. A method for producing a non-human animal carrying human herpcsviruses, comprising the steps of
a) crossbreeding a first non-human animal carrying human herpesviruses obtained according to the method of claim 9 with a second non-human animal belonging to the same species as said first animal but of a different sex, and
b) selecting the descendants carrying human herpesviruses.

11. A method according to claim 10, wherein said second animal is selected from a MOCK animal and a non-human animal carrying human herpesviruses.

## Patentansprüche

1. Verfahren zum Identifizieren einer potentiell therapeutischen Verbindung, umfassend die folgenden Schritte:
a) Infizieren eines nicht-menschlichen weiblichen Tieres mit einem menschlichem Herpesvirus;
b) Verabreichen der zu testenden potentiell therapeutischen Verbindung an das nicht-menschliche weibliche Tier;
c) Kreuzen des mit dem menschlichen Herpesvirus infizierten nicht-menschlichen weiblichen Tieres mit einem nicht-menschlichen männlichen Tier, das derselben Spezies wie das weibliche Tier angehört, und
d) Analysieren der Anwesenheit von menschlichen Herpesviren in den Nachkommen des mit einem menschlichen Herpesvirus infizierten nicht-menschlichen weiblichen Tieres und/oder Bestimmen der Wirkung der potentiell therapeutischen Verbindung auf die Nachkommen, wobei eine Verbindung als potentiell therapeutisch angesehen wird, wenn sie zu einer Abnahme der Virusbelastung in den Nachkommen führt und/oder wenn sie fähig ist, eines oder mehrere Symptome, die mit der Infektion mit menschlichen Herpesviren in Zusammenhang stehen, zu mildern oder aufzuheben, wobei die Symptome ausgewählt sind aus (1) Verhaltensänderung bei den Nachkommen, im Wesentlichen Reflexe, motorische und sensorische Fähigkeit sowie erkrankungsspezifische Symptome betreffend, wobei mithilfe einer Verhaltensanalyse ermittelt wird, (2) Strukturveränderungen auf cerebraler Ebene, (3) chromosomale Abweichungen, strukturelle genetische Veränderungen und Variation der Anzahl an Kopien und (4) neurodegenerative Prozesse,
wobei die Schritte a) und b) in beliebiger Reihenfolge ausgeführt werden.

2. Verfahren nach Anspruch 1, wobei Schritt a) vor Schritt b) ausgeführt wird.

3. Verfahren nach Anspruch 1, wobei Schritt b) vor Schritt a) ausgeführt wird.

4. Verfahren nach einem der Ansprüche 1 bis 3, wobei das nicht-menschliche Tier ein Fisch oder ein nicht-menschlicher Säuger ist.

5. Verfahren nach Anspruch 4, wobei der nicht-menschliche Säuger ausgewählt ist aus einem Nager, einem Primaten und einem Mitglied der Familie der Suidae.

6. Verfahren nach Anspruch 5, wobei der Nager ausgewählt ist aus Maus, Ratte und Hausmeerschweinchen.

7. Verfahren nach einem der Ansprüche 1 bis 3, wobei das menschliche Herpesvirus ausgewählt ist aus Herpes-simplex-Virus Typ 1 (HSV-1), Herpes-simplex-Virus Typ 2 (HSV-2), Varicella-Zoster-Virus (VZV), Cytomegalievirus (CMV), menschliches Herpesvirus 6 (HHV-6), menschliches Herpesvirus 7 (HHV-7), Epstein-Barr-Virus (EBV) und Kaposi-Sarkom assoziierte Herpesvirus (HHV-8) und Kombinationen davon.

8. Verfahren nach Anspruch 1, wobei die Bestimmung der Wirkung der getesten Verbindung auf die mit einem menschlichem Herpesvirus infizierten Nachkommen umfasst: das Analysieren der viralen Belastung und/oder das Durchführen mindestens einer Analyse oder Untersuchung ausgewählt aus: (1) Verhaltensanalyse, (2) bildgebenden Untersuchungsverfahren, (3) cytogenetischen Analysen und (4) neuropathologischen Untersuchungen.

9. Verfahren zum Erhalt eines nicht-menschlichen Tieres, das Träger menschlicher Herpesviren ist, umfassend die Schritte:
a) experimentelles Infizieren eines nicht-menschlichen weiblichen Tieres mit einem menschlichen Herpesvirus;
b) Kreuzen des mit einem menschlichem Herpesvirus infizierten nicht-menschlichen weiblichen Tieres mit einem nicht-menschlichen männlichen Tier, das derselben Spezies angehört wie das weibliche Tier, und
c) Selektieren der Nachkommen, die Träger menschlicher Herpesviren sind.

10. Verfahren zur Herstellung eines nicht-menschlichen Tieres, das Träger menschlicher Herpesviren ist, umfassend die Schritte:
a) Kreuzen eines ersten nicht-menschlichen Tieres, das Träger menschlicher Herpesviren ist und gemäß dem Verfahren nach Anspruch 9 erhalten wurde, mit einem zweiten nicht-menschlichen Tier, das derselben Spezies angehört wie das erste Tier aber ein anderes Geschlecht aufweist, und
b) Selektieren der Nachkommen, die Träger menschlicher Herpesviren sind.

11. Verfahren nach Anspruch 10, wobei das zweite Tier ausgewählt ist aus einem MOCK-Tier und einem nicht-menschlichen Tier, das Träger menschlicher Herpesviren ist.

## Revendications

1. Procédé d'identification d'un composé potentiellement thérapeutique, comprenant les étapes de :
a) infection d'un animal femelle non humain par un herpèsvirus humain ;
b) administration du composé potentiellement thérapeutique à tester audit animal femelle non humain ;
c) croisement dudit animal femelle non humain infecté par un herpèsvirus humain avec un animal mâle non humain appartenant à la même espèce que ladite femelle ; et
d) analyse de la présence d'herpèsvirus humains dans la descendance dudit animal femelle non humain infecté par un herpèsvirus humain et/ou détermination de l'effet dudit composé potentiellement thérapeutique sur ladite descendance, où un composé est considéré comme potentiellement thérapeutique s'il entraîne une diminution de la charge virale dans la descendance et/ou s'il est en mesure de diminuer ou de supprimer un ou plusieurs des symptômes associés à l'infection par les herpèsvirus humains où lesdits symptômes sont sélectionnés parmi (1) une modification du comportement de la descendance, essentiellement les réflexes, la capacité motrice et sensorielle de même que les symptômes spécifiques de la maladie, en utilisant l'analyse de comportement, (2) une modification structurelle au niveau cérébral, (3) des aberrations chromosomiques, des changements géné-tiques structurels et la variation du nombre de copies et (4) des processus neurodégénératifs,
où les étapes a) et b) sont mises en oeuvre dans un ordre quelconque.

2. Procédé selon la revendication 1, où l'étape a) est mise en oeuvre avant l'étape b).

3. Procédé selon la revendication 1, où l'étape b) est mise en oeuvre avant l'étape a).

4. Procédé selon l'une quelconque des revendications 1 à 3, où ledit animal non humain est un poisson ou un mammifère non humain.

5. Procédé selon la revendication 4, où ledit mammifère non humain est sélectionné parmi un rongeur, un primate et un suidé.

6. Procédé selon la revendication 5, où ledit rongeur est sélectionné parmi une souris, un rat et un cobaye commun.

7. Procédé selon l'une quelconque des revendications 1 à 3, où ledit herpèsvirus humain est sélectionné parmi le virus de l'herpès simplex de type 1 (HSV-1), le virus de l'herpès simplex de type 2 (HSV-2), le virus varicelle-zona (VZV), le cytomégalovirus (CMV), l'herpèsvirus humain 6 (HHV-6), l'herpèsvirus humain 7 (HHV-7), le virus Epstein-Barr (EBV) et l'herpèsvirus de Kaposi (HHV-8) et leurs mélanges.

8. Procédé selon la revendication 1, où la détermination de l'effet du composé testé sur ladite descendance infectée par un herpèsvirus humain comprend l'analyse de la charge virale et/ou la réalisation d'au moins une analyse ou étude sélectionnée parmi (1) une analyse de comportement, (2) des études d'imagerie, (3) des analyses cytogénétiques et (4) des études neuropathologiques.

9. Procédé d'obtention d'un animal non humain porteur d'herpèsvirus humains comprenant les étapes de :
a) infection expérimentale d'un animal femelle non humain par un herpèsvirus humain ;
b) croisement dudit animal femelle non humain infecté par un herpèsvirus humain avec un animal mâle non humain appartenant à la même espèce que ladite femelle, et
c) sélection des descendants porteurs d'herpèsvirus humains.

10. Procédé de production d'un animal non humain porteur d'herpèsvirus humains, comprenant les étapes de :
a) croisement d'un premier animal non humain porteur d'herpèsvirus humains obtenu selon le procédé de la revendication 9 avec un second animal non humain appartenant à la même espèce que ledit premier animal mais d'un sexe différent, et
b) sélection des descendants porteurs d'herpèsvirus humains.

11. Procédé selon la revendication 10, où ledit second animal est sélectionné parmi un animal MOCK et un animal non humain porteur d'herpèsvirus humains.
